# EUROPEAN PATENT SPECIFICATION

(11) **EP 0 890 574 B1**
(45) Date of publication and mention of the grant of the patent: **12.03.2003**
(21) Application number: 98111622.1
(22) Date of filing: 24.06.1998
(51) Int. Cl.: C07D 473/18, A61K 31/52

(54) **Method for purifying nucleic acid derivatives**
Verfahren zur Reindarstellung von Nukleinsäurederivaten
Procédé pour la purification de dérivés d'acide nucléique

(30) Priority: 10.07.1997 JP 18534397
(43) Date of publication of application: 13.01.1999
(73) Proprietor: Ajinomoto Co., Inc., Tokyo (JP)
(72) Inventor: Matsuzawa, Toshihiro, c/o Central Research Lab., Kanagawa-shi, Kanagawa-ken (JP); Nishiwaki, Tohru, c/o Central Research Lab., Kanagawa-shi, Kanagawa-ken (JP); Nishi, Seiichi, c/o Tokai Plant, Yokkaichi-shi, Mie-ken (JP); Yatagai, Masanobu, c/o Central Research Lab., Kanagawa-shi, Kanagawa-ken (JP)
(74) Representative: Strehl Schübel-Hopf & Partner

(56) References cited:
- EP-A- 0 184 473
- EP-A- 0 502 690
- EP-A- 0 675 123
- WO-A-97/18211
- MARTIN J C ET AL: "9-[(1,3-Dihydroxy-2-propoxy)methyl] guanine: a new potent and selective antiherpes agent" JOURNAL OF MEDICINAL CHEMISTRY, vol. 26, no. 5, May 1983, pages 759-761, XP000616048

## Description

### Field of the Invention

The present invention relates to a process for producing nucleic acid derivatives which are useful as an antiviral agent. More specifically, the present invention relates to a method for purifying nucleic acid derivatives obtained by alkylating a purine base, and intermediates in the purification thereof.

### Prior Art

(-)-9-[1'S, 2'R-bis(hydroxymethyl)cyclopropan-1'-yl]methylguanine represented by formula (II) 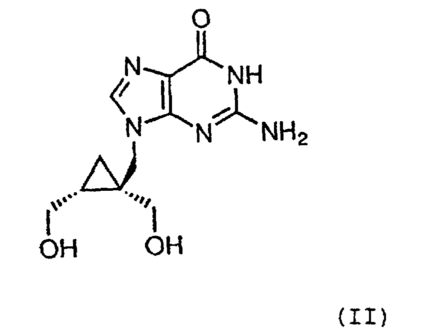 is known to have a high antiviral activity (Japanese Patent Laid-Open No. 78,357/1993).

The above-mentioned compound is produced by a process in which a protected purine base is alkylated as described in Japanese Patent Laid-Open Nos. 80,670/1994 and 316,155/1995. This alkylation occurs in both the 7- and 9-positions of the purine ring. In order to obtain a desired 9-substituted compound of the present invention, a 7-substituted compound and an unreacted purine base had to be separated through chromatography.

### Problems To Be Solved by the Invention

It is an object of the present invention to provide an industrial process for producing a compound represented by formula (II) in which an intricate procedure such as chromatography or the like is not conducted.

### Means For Solving the Problems

The present inventors have assiduously conducted investigations to solve the above-mentioned problems, and have consequently found that a desired 9-substituted compound represented by formula (I) can be obtained in high purity at good efficiency by reacting 2-amino-6-chloropurine with a compound obtained by substituting a hydroxyl group of (1S, 5R)-3-oxa-2-oxobicyclo[3.1.0]hexane-1-methanol with a leaving group, suspending or dissolving the resulting mixture of desired (-)-9-[1'S, 2'R-bis(hydroxymethyl)cyclopropan-1'-yl]methylguanine and by-product 7-[1'S, 2'R-bis(hydroxymethyl)cyclopropan-1'-yl)methylguanine in an alcohol or a hydrous alcohol, adding thereto any of an alkali metal hydroxide, an aqueous solution of an alkali metal hydroxide, an alcohol solution of an alkali metal hydroxide, an alkali metal alkoxide and an alcohol solution of an alkali metal alkoxide, then cooling the reaction mixture, and isolating crystals precipitated. This finding has led to the completion of the present invention.

That is, the present invention relates to a purification method which comprises suspending or dissolving a mixture of (-)-9-[1'S, 2'R-bis(hydroxymethyl)cyclopropan-1'-yl]methylg uanine and 7-[1'S, 2'R-bis(hydroxymethyl)cyclopropan-1'-yl] methylguanine in an alcohol or a hydrous alcohol, and reacting the suspension or the solution with an alkali metal hydroxide or alkoxide, and selectively precipitating crystals of nucleic acid derivatives represented by formula (I) 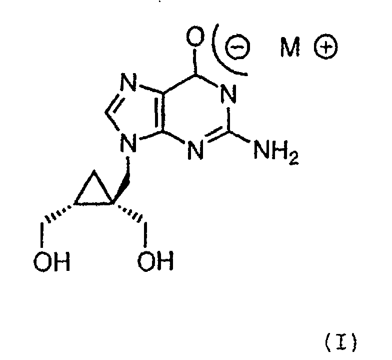 wherein M represents an alkali metal.

### Mode For Carrying Out the Invention

(-)-9-[1'S, 2'R-bis (hydroxymethyl)cyclopropan-1'-yl]me thylguanine represented by formula (II) which is a starting material of the present invention can be formed by the process described in Japanese Patent Laid-Open Nos. 80,670/1994 and 316,155/1995.

For example, 2-amino-6-chloropurine is reacted with 1-bromomethyl-4,4-diphenyl-3,5-dioxabicyclo[5,1,0]octane in an N,N-dimethylformamide solvent in the presence of potassium carbonate, and 2-amino-6-chloro-9-(4',4'-diphenyl-3',5'-dio xabicyclo[5,1,0]octyl-1'-)methylpurine obtained is hydrolyzed in formic acid through heating to form desired (-)-9-[1'S, 2'R-bis (hydroxymethyl)cyclopropan-1'-yl]methylguanine as an aqueous solution. This solution is concentrated to dryness, then dissolved in a sodium hydroxide aqueous solution, washed with ethyl acetate, and neutralized with hydrochloric acid to obtain desired (-)-9-[1'S, 2'R-bis(hydroxymethyl)cyclopropan-l'-yl]methylguranine as a crystal.

Alternatively, 2-amino-6-chloropurine is reacted with (3-oxa-2-oxobicyclo[3,1,0]hexan-1-yl)methylmethane sulfonate in N,N-dimethylformamide in the presence of potassium carbonate. 2-Amino-6-chloro-9-(3'-oxa-2'-oxobicyclo[3,1,0] hexan-1'-yl)methylpurine obtained is hydrolyzed with hydrochloric acid, and neutralized to give 9-(3'-oxa-2'-oxobicyclo[3,1,0]hexan-1'-yl]methylguanine as a crystal. This compound is reduced with sodium borohydride in an ethanol solvent, and treated with an acid. The thus-treated substance is subjected to substitution with a sodium hydroxide aqueous solution, and neutralized to obtain desired (-)-9-[1'S, 2'R-bis(hydroxymethyl)cyclopropan-1'-yl]methylg uanine as a crystal.

The crystals obtained by these methods contain, as mentioned above, desired (-)-9-[1'S, 2'R-bis(hydroxymethyl)cyclopropan-1'-yl)methylguanine and further 7-[1'S, 2'R-bis(hydroxymethyl)cyclopropan-1'-yl]methylguanine represented by the following formula (III) in which the 7-position of guanine is substituted, and guanine.

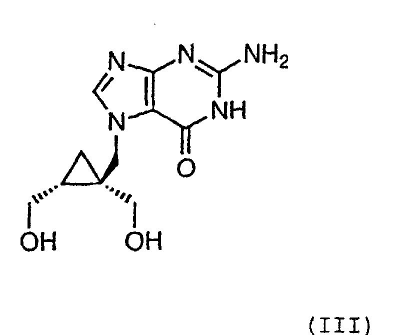

The desired compound represented by formula (I) in the present invention can be purified and produced from the mixture obtained in the above-mentioned methods in the following manner.

First, the starting compound (II) containing impurities such as 7-[1'S, 2'R-bis(hydroxymethyl)cyclopropan-1'-yl]met hylguanine and the like is suspended in an alcohol solvent such as methanol, ethanol or 2-propanol or in an alcohol solvent containing water at a volume ratio of from 0 to 50%. At this time, the concentration is preferably between 2 and 15% (w/w). Then, any of an alkali metal hydroxide, an aqueous solution of an alkali metal hydroxide, an alcohol solution of an alkali metal hydroxide, an alkali metal alkoxide and an alcohol solution of an alkali metal alkoxide in an amount of from 1 to 5 equivalents based on the compound (II) is added to this suspension. The reaction is conducted at from 0°C to a solvent reflux temperature for from 0.5 to 50 hours. Subsequently, the reaction mixture is cooled at from 0 to 30°C, preferably at from 0 to 10°C, and aged for from 0.5 to 50 hours to obtain the -desired compound of formula (I) as crystals. This compound can easily be isolated through filtration.

Alternatively, the starting compound (II) is dissolved in an alkaline aqueous solution (which is a sodium hydroxide aqueous solution, a potassium hydroxide aqueous solution or a lithium hydroxide aqueous solution), and an alcohol solvent such as methanol, ethanol or 2-propanol is added thereto. The desired compound of formula (I) is precipitated through cooling, and it can be obtained as crystals.

The compound of formula (I) in the present invention can also be represented by formula (I'). 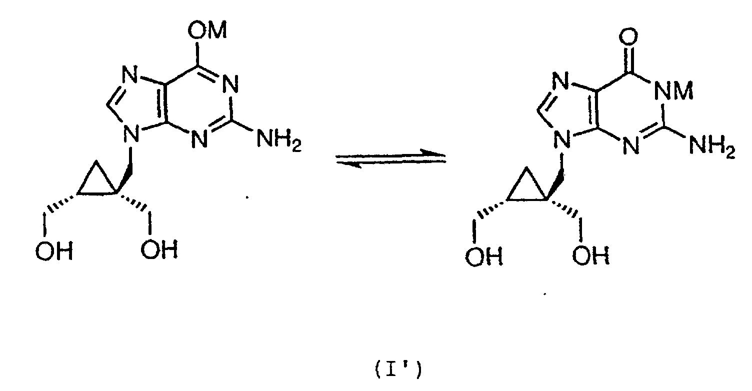 wherein M represents an alkali metal.

Examples of the alkali metal in the present invention include sodium, potassium and lithium. Examples of the alkali metal hydroxide include sodium hydroxide, potassium hydroxide and lithium hydroxide. Examples of the alkali metal alkoxide include solid compounds such as sodium methylate, sodium ethylate, sodium tert-butylate, potassium methylate and potassium tert-butylate; and corresponding alcohol solutions.

With respect to a preferable combination, there is a method in which a methanol solution of sodium methylate is added to a methanol suspension. The reason is that the reagent is easily obtainable at a low cost and also easy to handle, the resulting crystallinity is good and a high purity and a high yield are provided.

The crystal of the compound of formula (I) formed by the process of the present invention can be obtained in high purity, and ratios of isomer impurities and guanine which are hard to remove can be reduced to appreciable levels. The process of the present invention can also dispense with an intricate and economically disadvantageous procedure of purification with a silica gel column used to remove the above-mentioned impurities. Further, the compound of formula (I) can easily be converted to the compound of formula (II) through hydrolysis.

### Examples

The present invention is illustrated more specifically by referring to the following Examples.

### Example 1

### Production of (-)-9-[1'S, 2'R-bis (hydroxymethyl)cyclopropan-1'-yl]methylguanine sodium salt:

### <Process 1>

(-)-9-[1'S, 2'R-bis(hydroxymethyl)cyclopropan-1'-yl]me thylguanine was prepared according to the process described in Japanese Patent Laid-Open No. 80,670/1994 without conducting purification through silica gel column chromatography to obtain 44.8 g of a crude product. This product contained 63.6% of the desired product and 21.9% of a 7-position isomer.

This mixture was dissolved in 40 ml of a 4.5-N sodium hydroxide aqueous solution, and 730 ml of methanol were added thereto while being stirred at room temperature. The reaction solution was cooled for 2 hours while being stirred in an ice bath. Crystals precipitated were filtered, and washed with a small amount of hydrous methanol. The resulting crystals were dried at 70°C under reduced pressure for 3 hours to give 22.3 g of the above-mentioned compound. In the product, the content of the above-mentioned compound reached 88.9% (yield 64.2%), and the content of the 7-position isomer was decreased to 0.25%.

### <Process 2>

Unpurified (-)-9-[1'S, 2'R-bis (hydroxymethyl)cyclopropan-1'-yl]methylguanine was prepared by the process described in Japanese Patent Laid-Open No. 316,155/1995 to obtain 39.9 g of a crude product. The contents of the desired product, a 7-position isomer and guanine were 67.9%, 9.9% and 3.8% respectively. This mixture was suspended in methanol, and the suspension was stirred at 45°C with the addition of a methanol solution of sodium methoxide. After the mixture was stirred for 1 hour, the reaction solution was cooled to 10°C, and crystals precipitated were filtered, and washed with a small amount of methanol. The resulting crystals were dried at 50°C under reduced pressure to obtain 28 g of the above-mentioned compound as a single substance (content 94.4%, yield 90.2%).

### White crystal

¹H-NMR (300 MHz, CD₃OD) δ : 0.46 (t, J=5.5 Hz, 1H), 0.99 (dd, J=8.8, 5.2 Hz, 1H), 1.36 (tt, J=8.8, 6.2 Hz, 1H), 3.35 (d, J=12.6 Hz, 1H), 3.47 (dd, J=11.9, 8.8 Hz, 1H), 3.58 (d, J=12.6 Hz, 1H), 3.75 (dd, J=11.9, 6.5 Hz, 1H), 3.83 (d, J=14.4 Hz, 1H), 4.23 (d, J=14.9 Hz, 1H), 7.60 (s, 1H).

¹³C-NMR (75 MHz, CD₃OD) δ : 14.9, 25.6, 28.7, 49.3, 62.5, 62.8, 119.0, 138.0, 153.1, 162.1, 169.1.

Mass spectrum (FAB) 288 (MH⁺)

Melting point 220 - 230°C

### Example 2

### Production of (-)-9-[1'S, 2'R-bis(hydroxymethyl)cyclopropan-1'-yl]methylguanine potassium salt:

Unpurified (-)-9-[1'S, 2'R-bis(hydroxymethyl)cyclopropan-1'-yl]methylguanine (0.5g; contents of the desired product, a 7-position isomer and guanine were 85.7%, 5.3% and 3.2% respectively) obtained by the process described in Japanese Patent Laid-Open No. 316,155/1995 was suspended in 3 ml of methanol, and a potassium hydroxide methanol solution (0.23 g/3 ml) was added thereto while being stirred. After these were dissolved, crystals precipitated were filtered, and washed with a small amount of methanol. The resulting crystals were dried at 45°C under reduced pressure for 5 hours to obtain 0.35 g (yield 61.2%) of the above-mentioned compound. In the product, the content of the above-mentioned compound was 98.5%, and the contents of the 7-position isomer and guanine were 0.7% and 0.3% respectively.

### White crystal

¹H-NMR (300 MHz, CD₃OD) δ : 0.46 (t, J=5.4 Hz, 1H), 0.99 (dd, J=8.6, 5.2 Hz, 1H), 1.35 (tt, J=8.6, 6.1 Hz, 1H), 3.34 (d, J=12.4 Hz, 1H), 3.46 (dd, J=11.8, 8.8 Hz, 1H), 3.58 (d, J=12.6 Hz, 1H), 3.76 (dd, J=11.9, 6.4 Hz, 1H), 3.83 (d, J=14.6 Hz, 1H), 4.23 (d, J=14.6 Hz, 1H), 7.60 (s, 1H).

¹³C-NMR (75 MHz, CD₃OD) δ : 14.9, 25.6, 28.7, 49.3, 62.5, 62.7, 119.0, 138.0, 153.1, 162.1, 169.0.

Mass spectrum (ESI) 304 (MH⁺)

Melting point 186 - 188°C

### Example 3

### Production of (-)-9-[1'S, 2'R-bis (hydroxymethyl)cyclopropan-1'-yl]methylguanine lithium salt:

Unpurified (-)-9-[1'S, 2'R-bis(hydroxymethyl)cyclopropan-1'-yl]methylguanine (0.5 g; contents of the desired product, a 7-position isomer and guanine were 85.7%, 5.3% and 3.2% respectively) obtained by the process described in Japanese Patent Laid-Open No. 316,155/1995 was suspended in 6 ml of methanol, and 0.18 g of lithium hydroxide monohydrate was added thereto at room temperature while being stirred. After these were dissolved, crystals precipitated were filtered, and washed with a small amount of methanol. The resulting crystals were dried at 45°C under reduced pressure for 5 hours to give 0.48 g (yield 93.9%) of the above-mentioned compound. In the product, the content of the above-mentioned compound was 96.8%, and the contents of the 7-position isomer and guanine were 1.1% and 1.4% respectively.

### <Process 2>

Unpurified (-)-9-[1'S, 2'R-bis (hydroxymethyl) cyclopropan-1'-yl]methylguanine (0.5 g; contents of the desired product, a 7-position isomer and guanine were 85.7%, 5.3% and 3.2% respectively) obtained by the process described in Japanese Patent Laid-Open 316,155/1995 was suspended in 7 ml of ethanol, and 0.18 g of lithium hydroxide monohydrate and 0.5 ml of water were added thereto at room temperature while being stirred. Crystals precipitated were filtered, and washed with a small amount of ethanol. The resulting crystals were dried at 45°C under reduced pressure for 5 hours to give 0.5 g (yield 97.8%) of the above-mentioned compound. In the product, the content of the above-mentioned compound was 98.8%, and the contents of the 7-position isomer and guanine were 0.6% and 0.3% respectively.

### White crystal

¹H-NMR (300 MHz, CD₃OD) δ : 0.48 (t, J=5.3 Hz, 1H), 1.02 (dd, J=8.7, 4.9 Hz, 1H), 1.37 (m, 1H), 3.35 (--CD3OD--), 3.47 (dd, J=12.1, 8.7 Hz, 1H), 3.59 (d, J=12.5 Hz, 1H), 3.76 (dd, J=11.9, 6.5 Hz, 1H), 3.85 (d, J=14.6 Hz, 1H), 4.24 (d, J=14.3 Hz, 1H), 7.65 (s, 1H).

Mass spectrum (FAB) 272 (MH⁺)

Melting point 257°C (decomp.)

### Example 4

### Hydrolysis of (-)-9-[1'S, 2'R-bis(hydroxymethyl)cyclopropan-1'-yl]methylguanine sodium salt:

(-)-9-[1'S, 2'R-bis(hydroxymethyl)cyclopropan-1'-yl]me thylguanine sodium salt (21.7 g) prepared by <Process 2> of Example 1 was dissolved in 410 ml of water while being stirred. 3-N hydrochloric acid was added thereto until a pH of the solution reached a neutral value. The resulting suspension was stirred at 30°C for 2 hours. Subsequently, crystals precipitated were filtered, and washed with a small amount of water. The resulting crystals were dried overnight at 70°C under reduced pressure to give 18.8 g (content 96.8%, yield 96.5%) of high-purity (-)-9-[1'S, 2'R-bis(hydroxymethyl)cyclopropan-1'-yl]methylguanine. The properties thereof were completely consistent with those of the compound described in Japanese Patent Laid-Open No. 78,357/1993.

### [Effects of the Invention]

In accordance with the present invention, salts of nucleic acid derivatives having an antiviral activity can easily be produced at good efficiency, and a 7-position isomer and other impurities can effectively be separated during the production.

## Claims

1. A purification method which comprises suspending or dissolving a mixture comprising (-)-9-[1'S, 2'R-bis (hydroxymethyl)cyclopropan-1'-yl]methylguanine and (7-[1'S, 2'R-bis(hydroxymethyl)cyclopropan-1'-yl]methylguanine in an alcohol or a hydrous alcohol, and reacting the suspension or the solution with an alkali metal hydroxide or alkoxide, and selectively precipitating crystals of nucleic acid derivatives represented by formula (I) 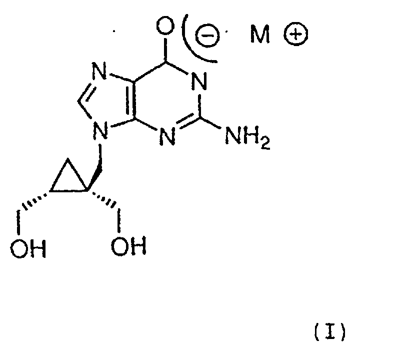 wherein M represents an alkali metal.

2. The purification method of claim 1, wherein the alkali metal hydroxide is sodium hydroxide.

3. The purification method of claim 1, wherein the alkali metal alkoxide is sodium alkoxide.

4. Nucleic acid derivatives represented by formula (I) 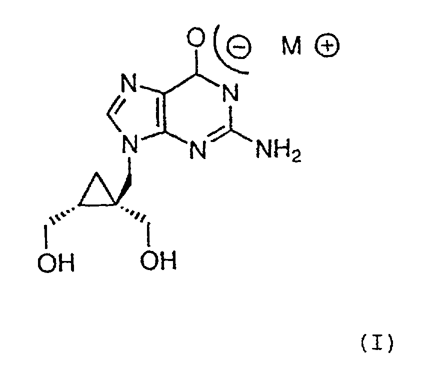 wherein M represents an alkali metal.

5. (-)-9-[1'S, 2'R-bis(hydroxymethyl)cyclopropan-1'-yl]methylguanine sodium salt.

## Patentansprüche

1. Reinigungsverfahren, welches das Suspendieren oder Auflösen eines Gemisches, das (-)-9-[1'S,2'R-Bis(hydroxymethyl)cyclopropan-1'-yl]methylguanin und (7-[1'S,2'R-Bis(hydroxymethyl)cyclopropan-1'-yl]methylguanin enthält, in einem Alkohol oder einem wässerigen Alkohol und das Umsetzen der Suspension oder der Lösung mit einem Alkalimetallhydroxid oder Alkoxid und das selektive Ausfällen von Kristallen von Nukleinsäurederivaten der Formel (I) 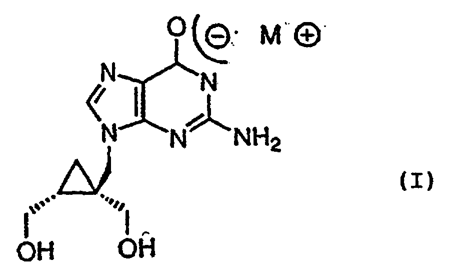 umfaßt, worin M ein Alkalimetall darstellt.

2. Reinigungsverfahren nach Anspruch 1, wobei das Alkalimetallhydroxid Natriumhydroxid ist.

3. Reinigungsverfahren nach Anspruch 1, wobei das Alkalimetallalkoxid Natriumalkoxid ist.

4. Nukleinsäurederivat der Formel (I) 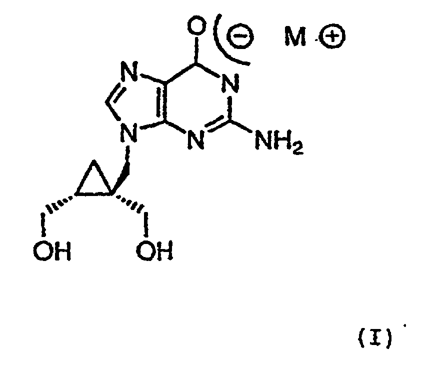 worin M ein Alkalimetall darstellt.

5. (-)-9-[1'S,2'R-Bis(hydroxymethyl)cyclopropan-1'yl]methylguanin-Natriumsalz.

## Revendications

1. Procédé de purification qui comprend la mise en suspension ou la dissolution d'un mélange constitué de (-)-9-[1'S, 2'R-bis(hydroxyméthyle)cyclopropane-1'-yl]méthylguanine et (7-[1'S, 2'R-bis(hydroxyméthyle)-cyclopropane-1'-yl]méthylguanine dans un alcool ou un alcool aqueux, et la réaction de la suspension ou de la solution avec un hydroxyde ou alcoolate métallique alcalin, et la précipitation sélective de cristaux de dérivés d'acides nucléiques représentés par la formule (I) 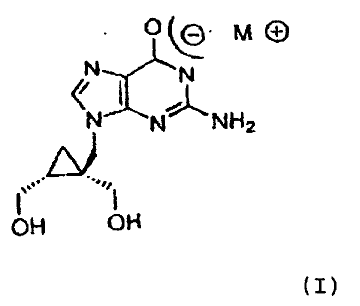 dans laquelle M représente un métal alcalin.

2. Procédé de purification selon la revendication 1,
dans lequel l'hydroxyde métallique alcalin représente l'hydroxyde de sodium.

3. Procédé de purification selon la revendication 1,
dans lequel l'alcoolate métallique alcalin représente l'alcoolate de sodium.

4. Dérivés d'acides nucléiques représentés par la formule (I) 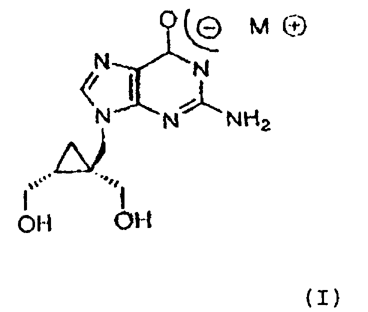 dans laquelle M représente un métal alcalin.

5. Sel de sodium (-)-9-[1'S, 2'R-bis(hydroxyméthyle)cyclopropane-1'-yl]méthylguanine.
